(19) Europäisches Patentamt
European Patent Office
Office européen des brevets

(11) **EP 2 533 747 B1**

(12) **EUROPÄISCHE PATENTSCHRIFT**

(45) Veröffentlichungstag und Bekanntmachung des Hinweises auf die Patenterteilung:
**22.06.2022 Patentblatt 2022/25**

(21) Anmeldenummer: **11707565.5**

(22) Anmeldetag: **18.01.2011**

(51) Internationale Patentklassifikation (IPC):
**A61H 23/02** (2006.01)  **A61H 7/00** (2006.01)

(52) Gemeinsame Patentklassifikation (CPC):
**A61H 23/0245; A61H 7/001; A61H 23/0254;**
A61H 2201/10; A61H 2201/1418; A61H 2201/1669;
A61H 2201/5002; A61H 2201/5084; A61H 2205/06;
A61H 2205/065; A61H 2205/10; A61H 2205/12;
A61H 2230/10; A61H 2230/30; A61H 2230/40;

(Forts.)

(86) Internationale Anmeldenummer:
**PCT/DE2011/075004**

(87) Internationale Veröffentlichungsnummer:
**WO 2011/098082 (18.08.2011 Gazette 2011/33)**

(54) **VORRICHTUNG ZUR BEHANDLUNG EINES PATIENTEN MIT VIBRATIONS-, TAST- UND/ODER THERMOREIZEN**

APPARATUS FOR TREATING A PATIENT WITH VIBRATORY, TACTILE AND/OR THERMAL STIMULI

APPAREIL POUR LE TRAITEMENT D'UN PATIENT PAR L'APPLICATION DE STIMULATIONS VIBRATOIRES, TACTILES ET/OU THERMALES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.02.2010 DE 102010000390**

(43) Veröffentlichungstag der Anmeldung:
**19.12.2012 Patentblatt 2012/51**

(73) Patentinhaber: Tass, Peter
**83684 Tegernsee (DE)**

(72) Erfinder:
• **TASS, Peter, Alexander**
**81541 München (DE)**
• **MAYOR, Laetitia**
**CH-1723 Marly (CH)**
• **ROULET, Jean-Christophe**
**CH-2523 Ligniéres (CH)**
• **SCHNELL, Urban**
**CH-3053 Münchenbuchsee (CH)**

(74) Vertreter: **Dörries, Hans Ulrich**
**df-mp Dörries Frank-Molnia & Pohlman Patentanwälte Rechtsanwälte PartG mbB Theatinerstrasse 16 80333 München (DE)**

(56) Entgegenhaltungen:
EP-A2- 2 103 288    WO-A1-2009/136931
CA-A1- 2 623 464    US-A1- 2007 232 962
US-A1- 2007 255 187

(52) Gemeinsame Patentklassifikation (CPC): (Forts.)
A61H 2230/60; A61H 2230/65

**Beschreibung**

Die Erfindung betrifft eine Vorrichtung zur Behandlung eines Patienten mit Vibrations- und/oder Tast- und/oder Thermoreizen.

Insbesondere bezieht sich die Erfindung auf eine Stimulationsvorrichtung zur Behandlung von Erkrankungen, bei denen eine gesteigerte neuronale Synchronisation vorliegt. Mit der Erfindung können insbesondere Erkrankungen des Gehirns behandelt werden, wie z.B. Bewegungsstörungen, Morbus Parkinson, essentieller Tremor, Dystonie, Migräne, Spannungskopfschmerzen, Spastik, Funktionsstörungen nach Schlaganfall, neuropathische Schmerzen, chronische Schmerzzustände, Neuralgien, Amputationsschmerzen, Tremor und andere Funktionsstörungen bei Zustand nach Hirntrauma. Es können aber auch gastrointestinale Erkrankungen, wie z.B. das Reizdarm-Syndrom, behandelt werden. Hierbei können schmerzhafte Verkrampfungen und ineffiziente Darmmotilität verlernt werden. Auch bei der Colitis ulcerosa und beim Morbus Crohn kann die Behandlung mit der erfindungsgemäßen Vorrichtung krampflösend und schmerzlindernd wirken. Des Weiteren können Asthma bronchiale, kardiale Ischämien sowie die periphere arterielle Verschlusskrankheit mit der erfindungsgemäßen Vorrichtung behandelt werden.

Zur Behandlung der vorstehend genannten Krankheiten gibt es - wenn überhaupt - nur pharmakologische oder stereotaktische Therapien. Die Effektivität der pharmakologischen Therapien ist typischerweise zeitlich begrenzt. Die stereotaktische Behandlung birgt relevante Risiken, z.B. das Risiko einer arteriellen Blutung bei der Implantation der Hirnschrittmacher. Aus den Schriften US 2007 / 0 255 187 A1, WO 2009 / 136 931 A1, US 2007 / 0 232 962 A1 und CA 2 623 464 A1 sind Vorrichtungen zur Behandlung eines Patienten mit Vibrations- und/oder Tast- und/oder Thermoreizen bekannt. EP 2 103 288 A2 offenbart eine Vorrichtung zur auditorischen Stimulation, die mehrere akustische Stimulationssignale unterschiedlicher Frequenzen erzeugt. Die Stimulationssignale sind derart gestaltet, dass diese über Hörnerven im Innenohr eines Patienten aufgenommen und an eine Neuronenpopulation im auditorischen Cortex des Patienten weitergeleitet werden. Die unterschiedlichen Stimulationssignale sind so gewählt, dass unterschiedliche Subpopulationen der Neuronenpopulation angesprochen werden.

Vor diesem Hintergrund wird eine Vorrichtung gemäß Anspruch 1 angegeben. Eine vorteilhafte Weiterbildung und Ausgestaltung der Erfindung ist im Anspruch 2 genannt.

Die Erfindung wird nachfolgend in beispielhafter Weise unter Bezugnahme auf die Zeichnungen näher erläutert. In diesen zeigen:

Fig. 1          eine schematische Darstellung einer Vorrichtung 100 zur Erzeugung von Vibrations-, Tast- und/oder Thermoreizen gemäß einem Ausführungsbeispiel;

Fig. 2          eine schematische Darstellung eines mit der Vorrichtung 100 ausgeführten Stimulationsverfahrens;

Fig. 3A bis 3D     schematische Darstellungen von Vibrationsreizen;

Fig. 4          eine schematische Darstellung eines Tastreizes;

Fig. 5A bis 5C     schematische Darstellungen von Thermoreizen;

Fig. 6          eine schematische Darstellung der Vorrichtung 100 während ihres bestimmungsgemäßen Betriebs;

Fig. 7 bis 9     schematische Darstellungen von mit der Vorrichtung 100 ausgeführten Stimulationsverfahren;

Fig. 10         eine schematische Darstellung einer Vorrichtung 200 zur Erzeugung von Vibrations-, Tast- und/oder Thermoreizen gemäß einem weiteren Ausführungsbeispiel;

Fig. 11A bis 13C   schematische Darstellungen von Stimulationseinheiten zur Erzeugung von Vibrations- und/oder Tastreizen;

Fig. 14A bis 15C   schematische Darstellungen von Stimulationseinheiten zur Erzeugung von Thermoreizen;

Fig. 16 und 17     schematische Darstellungen von Stimulationsverfahren; und

Fig. 18 bis 19C    schematische Darstellungen einer Vorrichtung 300 zur Erzeugung von Vibrations-, Tast- und/oder Thermoreizen gemäß einem weiteren Ausführungsbeispiel.

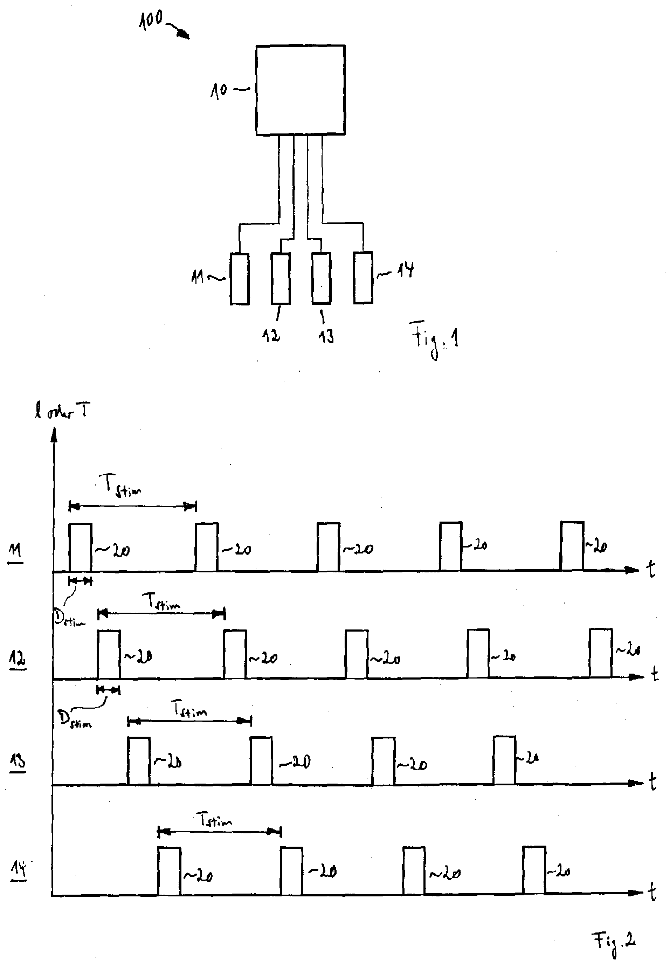

In Fig. 1 ist schematisch eine Vorrichtung 100 zur nichtinvasiven Behandlung eines Patienten mit Vibrations-, Tast- und/oder Thermoreizen dargestellt. Die Vorrichtung 100 besteht in der in Fig. 1 gezeigten Ausgestaltung aus einer ersten Stimulationseinheit 11 zur Erzeugung erster Reize, einer zweiten Stimulationseinheit 12 zur Erzeugung zweiter Reize, einer dritten Stimulationseinheit 13 zur Erzeugung dritter Reize und einer vierten Stimulationseinheit 14 zur Erzeugung vierter Reize. Die in Fig. 1 gezeigte Ausgestaltung ist lediglich beispielhaft zu verstehen. Alternativ zu dieser Ausgestaltung kann die Vorrichtung 100 eine beliebige Anzahl N (N = 2, 3, 4, 5, 6, 7, 8, 9, 10, 11, 12,...) von Stimulationseinheiten enthalten. Die Vorrichtung 100 kann ferner eine Steuereinheit 10 aufweisen, die über geeignete Verbindungsleitungen oder über Funk mit den Stimulationseinheiten 11 bis 14 verbunden ist und die Erzeugung der Reize steuert. Die Steuereinheit 10 kann auch in eine oder mehrere oder alle Stimulationseinheiten 11 bis 14 integriert sein.

Die Stimulationseinheiten 11 bis 14 können jeweils einen oder mehrere Reize aus der Gruppe der Vibrations-, Tast- und Thermoreize erzeugen. Die Stimulationseinheiten 11 bis 14 sind derart ausgestaltet, dass sie auf die Haut des Patienten aufgesetzt werden können. Je nach Erkrankung bzw. betroffenen Körperpartien werden die Stimulationseinheiten 11 bis 14 in einer geeigneten Anordnung auf der Haut des Patienten befestigt, beispielsweise am Arm, am Bein, an der Hand und/oder am Fuß des Patienten. Vibrations-, Tast- und Thermostimulation können je nach Krankheitsbild entweder einzeln oder in Kombination auf der Haut verabreicht werden.

Die Mehrzahl von Stimulationseinheiten 11 bis 14 ermöglicht es, unterschiedliche rezeptive Bereiche der Haut über die einzelnen Stimulationseinheiten 11 bis 14 zeitlich und räumlich koordiniert zu stimulieren. Die Stimulationseinheiten 11 bis 14 können so auf der Haut des Patienten angeordnet sein, dass die auf das Hautgewebe applizierten Reize über Nervenleitungen an unterschiedliche Zielgebiete, die z.B. im Rückenmark und/oder im Gehirn liegen, weitergeleitet werden. Folglich können mittels der Vorrichtung 100 verschiedene Zielgebiete im Rückenmark und/oder Hirn während desselben Stimulationszeitraums mit eventuell unterschiedlichen und/oder zeitversetzten Reizen stimuliert werden.

Ein Stimulationsverfahren, das mit der Vorrichtungen 100 durchgeführt werden kann, ist in Fig. 2 schematisch dargestellt. In Fig. 2 sind untereinander die über die Stimulationseinheiten 11 bis 14 applizierten Reize 20 gegen die Zeit t aufgetragen.

Bei dem in Fig. 2 dargestellten Verfahren appliziert jede der Stimulationseinheiten 11 bis 14 den Reiz 20 periodisch an den jeweiligen rezeptiven Bereich der Haut, auf dem die Stimulationseinheit 11 bis 14 angebracht ist. Die Frequenz $f_{stim} = 1/T_{stim}$ ($T_{stim}$ = Periodendauer), mit welcher die von jeder der Stimulationseinheiten 11 bis 14 erzeugten Reize 20 wiederholt werden, kann im Bereich von 1 bis 60 Hz und insbesondere im Bereich von 30 bis 60 Hz oder im Bereich von 1 bis 30 Hz oder im Bereich 1 bis 20 Hz oder im Bereich von 5 bis 20 Hz liegen, kann aber auch kleinere oder größere Werte annehmen. Die Dauer $D_{stim}$ eines einzelnen Reizes 20 kann insbesondere von der Art des Reizes abhängen. Die in Fig. 2 gezeigte Ordinate hängt ebenfalls von der Art der Reize 20 ab. Bei einem Vibrations- oder Tastreiz kann beispielsweise die Auslenkung I eines Stimulationselements gegen die Zeit t aufgetragen werden, bei einem Thermoreiz kann eine Temperatur T dargestellt werden. Die über die verschiedenen Stimulationseinheiten 11 bis 14 applizierten Reize 20 können identisch oder aber verschieden sein.

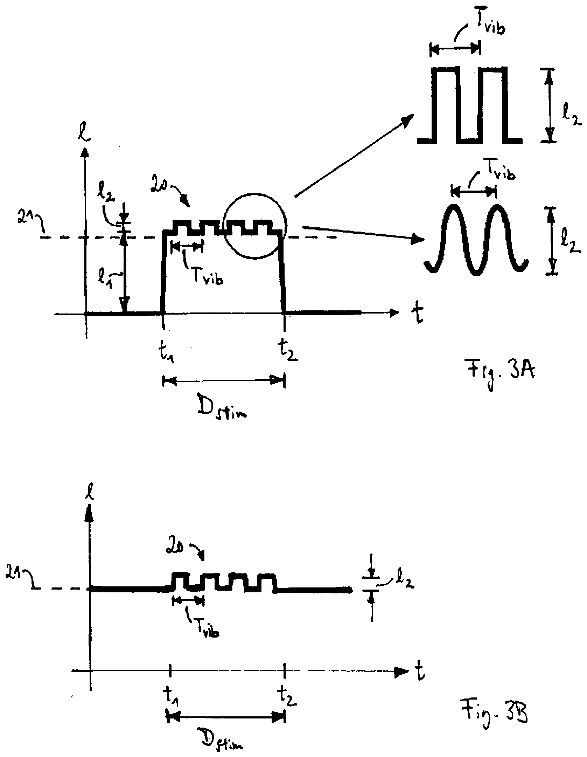

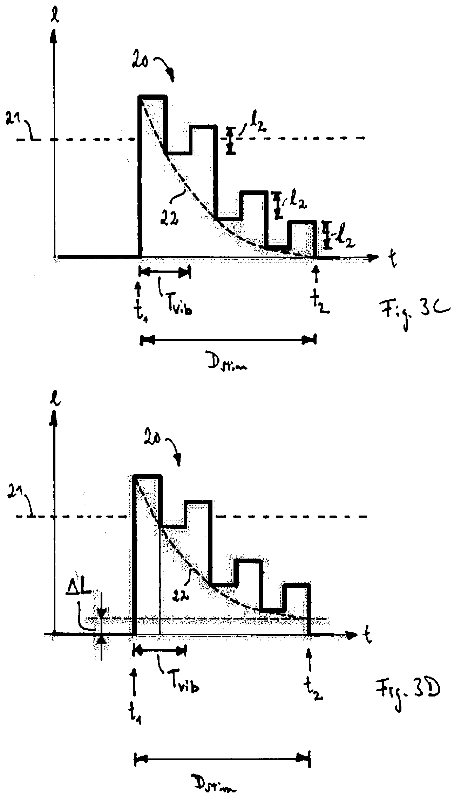

Verschiedene Ausgestaltungen einzelner Vibrationsreize 20 sind in Fig. 3A, 3B, 3C und 3D dargestellt. Dort ist die Auslenkung I eines Stimulationselements gegen die Zeit t aufgetragen. In Fig. 3A wird das Stimulationselement zur Zeit $t_1$ aus seiner Ruheposition ausgelenkt und in die Haut des Patienten eingedrückt. Die Lage der Hautoberfläche ist durch eine gestrichelte Linie 21 dargestellt. Nachdem das Stimulationselement in Kontakt mit der Haut getreten ist, wird ein periodischer Vibrationsreiz mit einer Frequenz $f_{vib} = 1/T_{vib}$ im Bereich von 30 bis 300 Hz appliziert ($T_{vib}$ = Periodendauer des Vibrationsreizes). Bei einer Frequenz $f_{vib}$ von 300 Hz kann das Stimulationselement eine Kraft von etwa 2 N ausüben. Die Dauer $D_{stim}$ des Vibrationsreizes 20 kann im Bereich von 10 bis 500 ms liegen. Insbesondere liegt die Stimulationsdauer $D_{stim}$ im Bereich von

$$0 < D_{stim} < \frac{T_{stim}}{N}, \qquad\qquad (1)$$

wobei N die Anzahl der Stimulationseinheiten ist. Z.B. ergibt sich für $T_{stim}$ = 1 Hz und N = 4 ein Bereich von 10 bis 250 ms für die Stimulationsdauer $D_{stim}$. Es können aber auch zeitlich überlappende Stimuli verwendet werden.

Zur Zeit $t_2$ wird das Stimulationselement wieder in seine Ruheposition verbracht, wo es keinen Kontakt zur Haut hat. Wie in Fig. 3A gezeigt kann der Vibrationsreiz 20 ein rechteckförmiger oder sinusförmiger Reiz sein, er kann aber auch andere Formen haben. Die in Fig. 3A gezeigte Auslenkung $I_1$ zur Eindrückung des Stimulationselements in die Haut kann im Bereich von 0,5 bis 3 mm liegen. Die Auslenkung $I_2$ des Stimulationselements während der Vibration kann zwischen 0,1 und 0,5 mm betragen.

In Fig. 3B ist eine Variation des in Fig. 3A gezeigten Vibrationsreizes 20 dargestellt. Bei der in Fig. 3B gezeigten Ausgestaltung steht das Stimulationselement stets in Kontakt mit der Haut des Patienten. Während des Stimulationszeitraums $D_{stim}$ wird ein wie oben beschriebener Vibrationsreiz 20 appliziert.

Eine weitere Variation des Vibrationsreizes 20 ist in Fig. 3C dargestellt. Im Unterschied zum Vibrationsreiz 20 aus Fig. 3A wird das Stimulationselement bereits während des Stimulationszeitraums $D_{stim}$ wieder zurückgefahren, so dass die Vibrationen mit zunehmender Zeitdauer weniger in die Haut eindrücken und sich das Stimulationselement schließlich vollständig von der Haut löst. Beispielsweise kann das Zurückfahren des Stimulationselements entlang einer linearen oder nichtlinearen, z.B. exponentiellen, Kurve 22 erfolgen, welcher die Vibrationen $f_{vib}$ des Stimulationselements überlagert sind. In dem in Fig. 3C gezeigten Beispiel reicht die abfallende Flanke eines jeden Pulses bis auf die Kurve 22 herunter. Der sich daran anschließende Puls hat eine fest vorgegebene Höhe $l_2$, d.h. die ansteigende Flanke eines jeden Pulses hat die Höhe $l_2$.

Eine Variation des Vibrationsreizes 20 aus Fig. 3C ist in Fig. 3D gezeigt. Dort geht die Kurve 22 nicht bis auf die Nulllinie ($l = 0$) zurück, sondern hat einen fest vorgegebenen Offset $\Delta L$ von der Nulllinie.

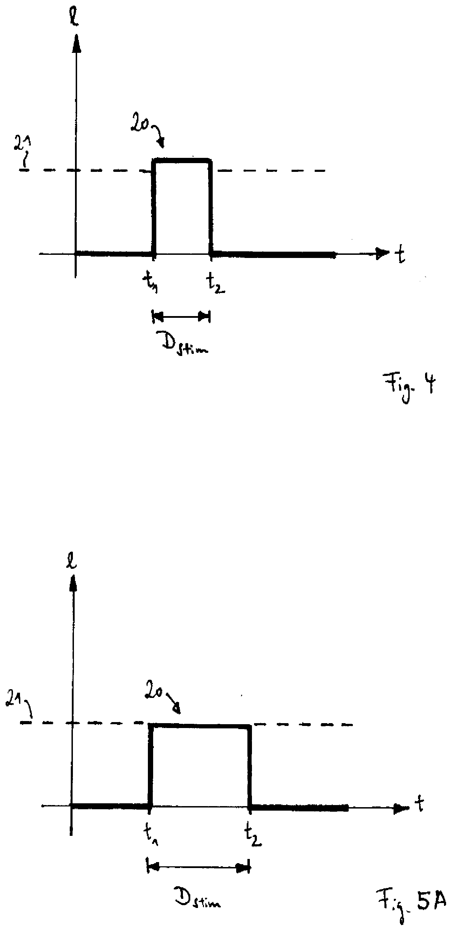

Eine Ausführungsform eines Tastreizes 20 ist in Fig. 4 gezeigt. Das Stimulationselement wird zur Zeit $t_1$ in die Haut des Patienten eingedrückt, verweilt dort für die Stimulationsdauer $D_{stim}$ und wird zur Zeit $t_2$ wieder zurückgefahren. Die Stimulationsdauer $D_{stim}$ liegt bei einem Tastreiz 20 im Bereich von 10 bis 500 ms. Insbesondere liegt die Stimulationsdauer $D_{stim}$ in dem oben in (1) angegebenen Bereich, es können aber auch zeitlich überlappende Stimuli verwendet werden.

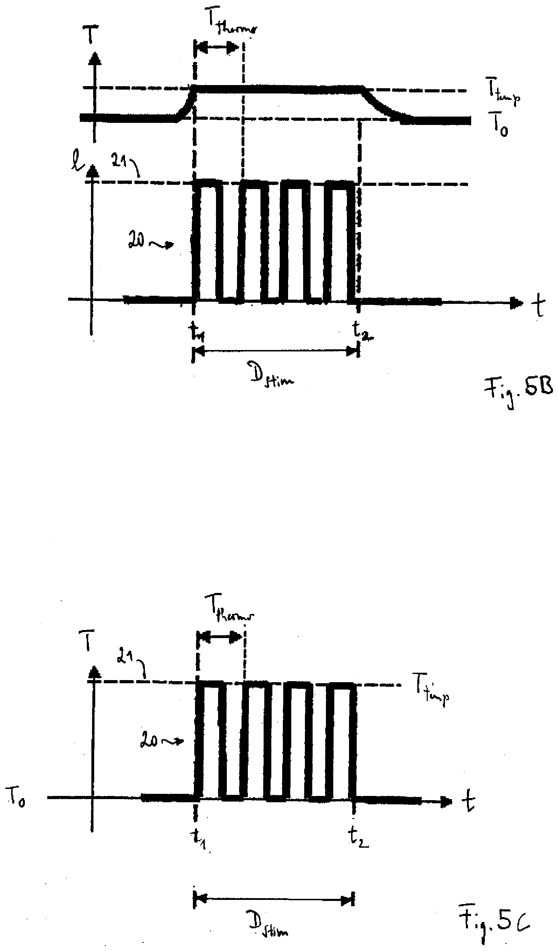

Verschiedene Ausführungsformen einzelner Thermoreize 20 sind in Fig. 5A, 5B und 5C dargestellt. Bei den in Fig. 5A und 5B gezeigten Ausgestaltungen wird ein Stimulationselement auf eine Temperatur $T_{temp}$ erhitzt oder gekühlt. Wie in Fig. 5B gezeigt ist, kann die Temperatur $T_{temp}$ erst kurz vor der Applikation des Thermoreizes 20 erzeugt werden. In diesem Fall hat das Stimulationselement während der Stimulationspausen eine Temperatur $T_0$, welche z.B. der Raumtemperatur entspricht. Alternativ kann das Stimulationselement auf einer konstanten Temperatur $T_{temp}$ gehalten werden.

Bei der Ausgestaltung nach Fig. 5A wird das erhitzte oder gekühlte Stimulationselement zur Zeit $t_1$ auf die Haut des Patienten gebracht und verbleibt dort für die gesamte Stimulationsdauer $D_{stim}$. Im Unterschied dazu wird bei der Ausgestaltung nach Fig. 5B das Stimulationselement während der Stimulationsdauer $D_{stim}$ periodisch mit einer Frequenz $f_{thermo}$ zur Haut verbracht und wieder entfernt. Die Frequenz $f_{thermo} = 1/T_{thermo}$ kann im Bereich von 1 bis 10 Hz liegen ($T_{thermo}$ = Periodendauer des Thermoreizes).

Der in Fig. 5C gezeigte Thermoreiz 20 entspricht im Wesentlichen dem Thermoreiz 20 aus Fig. 5B. Der Unterschied ist, dass der Thermoreiz 20 aus Fig. 5C berührungslos erzeugt wird. Hier wird die Stimulationstemperatur $T_{temp}$ durch elektromagnetische Strahlung, beispielsweise Infrarotlicht, erzeugt. Ferner wird die elektromagnetische Strahlung periodisch mit der Frequenz $f_{thermo} = 1/T_{thermo}$ variiert (z.B. durch An- und Ausschalten eines Infrarotstrahlers).

Bei Thermoreizen liegt die Stimulationsdauer $D_{stim}$ im Bereich von 10 bis 500 ms. Insbesondere liegt die Stimulationsdauer $D_{stim}$ in dem oben in (1) angegebenen Bereich, es können aber auch zeitlich überlappende Stimuli verwendet werden. Die Temperatur $T_{temp}$ kann von 22 bis 42 °C betragen. Die Temperatur $T_0$ ist in der Regel die Körpertemperatur des Patienten. Die Frequenz $f_{thermo}$ kann zwischen 1 und 10 Hz liegen, kann aber auch außerhalb dieses Bereichs liegen.

Es ist auch denkbar, dass ein einzelner Reiz 20 mehrere Reizarten umfasst. Beispielsweise kann der in Fig. 3A gezeigte Vibrationsreiz 20 gleichzeitig ein Thermoreiz sein, sofern das den Reiz ausübende Stimulationselement entsprechend erwärmt oder gekühlt ist. Ferner ist der Vibrationsreiz 20 aus Fig. 3A gleichzeitig ein Tastreiz (durch das Auftreffen des Stimulationselement auf die Haut werden Tastrezeptoren aktiviert).

Die Vorrichtung 100 kann insbesondere zur Behandlung von Erkrankungen, bei denen eine gesteigerte neuronale Synchronisation vorliegt, eingesetzt werden. Mit der Vorrichtung 100 können insbesondere Erkrankungen des Gehirns behandelt werden, wie z.B. Bewegungsstörungen, Morbus Parkinson, essentieller Tremor, Dystonie, Migräne, Spannungskopfschmerzen, Spastik, Funktionsstörungen nach Schlaganfall, neuropathische Schmerzen, chronische Schmerzzustände, Neuralgien, Amputationsschmerzen, Tremor und andere Funktionsstörungen bei Zustand nach Hirntrauma. Es können aber auch gastrointestinale Erkrankungen, wie z.B. das Reizdarm-Syndrom, behandelt werden. Des Weiteren können Colitis ulcerosa, Morbus Crohn, Asthma bronchiale, kardiale Ischämien sowie die periphere arterielle Verschlusskrankheit mit der Vorrichtung 100 behandelt werden.

Die vorstehend genannten Krankheiten können durch eine Störung der bioelektrischen Kommunikation von Neuronenverbänden, die in spezifischen Schaltkreisen zusammengeschlossen sind, verursacht werden. Hierbei generiert eine Neuronenpopulation anhaltend krankhafte neuronale Aktivität und möglicherweise eine damit verbundene krankhafte Konnektivität (Netzwerkstruktur). Dabei bilden eine große Anzahl von Neuronen synchron Aktionspotentiale aus, d.h. die beteiligten Neuronen feuern übermäßig synchron. Hinzu kommt, dass die kranke Neuronenpopulation eine oszillatorische neuronale Aktivität aufweist, d.h. die Neuronen feuern rhythmisch. Im Fall von neurologischen oder psychiatrischen Erkrankungen liegt die mittlere Frequenz der krankhaften rhythmischen Aktivität der betroffenen Neuronenverbände etwa im Bereich von 1 bis 30 Hz, kann aber auch außerhalb dieses Bereichs liegen. Bei gesunden Menschen feuern die Neuronen hingegen qualitativ anders, z.B. auf unkorrelierte Weise.

Die von den Stimulationseinheiten 11 bis 14 applizierten Reize 20 werden von in oder unter der Haut gelegenen Rezeptoren aufgenommen und an das Nervensystem weitergeleitet. Zu diesen Rezeptoren zählen beispielsweise Merkel-Zellen, Ruffini-Körperchen, Meissner-Körperchen und Haarfollikelrezeptoren, die insbesondere als Rezeptoren für die Tastreize 20 wirken. Die Vibrationsreize 20 zielen vorwiegend auf die Tiefensensibilität ab. Die Vibrationsreize 20 können von in der Haut, den Muskeln, dem Subkutangewebe und/oder den Sehnen des Patienten gelegenen Rezeptoren aufgenommen werden. Als Rezeptoren für die Vibrationsreize seien beispielhaft die Vater-Pacini-Körperchen genannt, die Vibrationsempfindungen und Beschleunigungen vermitteln. Die Thermoreize werden von den Thermorezeptoren der Haut aufgenommen. Dies sind Warmrezeptoren (auch Wärmerezeptoren, Warmsensoren oder Wärmesensoren genannt) und Kaltsensoren (auch Kältesensoren, Kaltrezeptoren oder Kälterezeptoren genannt). In der Haut des Menschen liegen die Kaltsensoren mehr oberflächlich, die Warmrezeptoren etwas tiefer.

Die von den Stimulationselementen 11 bis 14 generierten Reize 20 sind derart ausgestaltet, dass sie, wenn sie von den entsprechenden Rezeptoren aufgenommen werden und über die Nervenleitungen zu einer Neuronenpopulation im Gehirn oder Rückenmark mit einer krankhaft synchronen und oszillatorischen Aktivität geleitet werden, in der Neuronenpopulation ein Zurücksetzen, einen sogenannten Reset, der Phase der neuronalen Aktivität der stimulierten Neuronen bewirken. Durch das Zurücksetzen wird die Phase der stimulierten Neuronen unabhängig von dem aktuellen Phasenwert auf einen bestimmten Phasenwert, z.B. 0°, gesetzt. Somit wird die Phase der neuronalen Aktivität der krankhaften Neuronenpopulation mittels einer gezielten Stimulation kontrolliert.

Ferner ist es aufgrund der Mehrzahl von Stimulationselementen möglich, die krankhafte Neuronenpopulation an unterschiedlichen Stellen zu stimulieren. Die an unterschiedlichen Stellen der Haut applizierten Reize 20 werden nämlich an unterschiedliche Stellen im Gehirn oder Rückenmark weitergeleitet. Dies ermöglicht es, die Phase der neuronalen Aktivität der krankhaften Neuronenpopulation an den unterschiedlichen Stimulationsstellen zu unterschiedlichen Zeitpunkten zurückzusetzen. Im Ergebnis wird dadurch die krankhafte Neuronenpopulation, deren Neuronen zuvor synchron und mit gleicher Frequenz und Phase aktiv waren, in mehrere Subpopulationen aufgespalten. Innerhalb einer Subpopulation sind die Neuronen weiterhin synchron und feuern auch weiterhin mit derselben pathologischen Frequenz, aber jede der Subpopulationen weist bezüglich ihrer neuronalen Aktivität die Phase auf, die ihr durch den Stimulationsreiz aufgezwungen wurde.

Bedingt durch die krankhafte Interaktion zwischen den Neuronen ist der durch die Stimulation erzeugte Zustand mit mindestens zwei Subpopulationen instabil, und die gesamte Neuronenpopulation nähert sich schnell einem Zustand kompletter Desynchronisation, in welchem die Neuronen unkorreliert feuern. Der gewünschte Zustand, d.h. die komplette Desynchronisation, ist somit nach der Applikation der Reize 20 nicht sofort vorhanden, sondern stellt sich meist innerhalb weniger Perioden oder gar in weniger als einer Periode der pathologischen Aktivität ein.

Bei der vorstehend beschriebenen Art der Stimulation wird die letztlich gewünschte Desynchronisation durch die krankhaft gesteigerte Interaktion zwischen den Neuronen erst ermöglicht. Hierbei wird ein Selbstorganisationsprozess ausgenutzt, der für die krankhafte Synchronisation verantwortlich ist. Derselbe bewirkt, dass auf eine Aufteilung einer Gesamtpopulation in Subpopulationen mit unterschiedlichen Phasen eine Desynchronisation folgt. Im Gegensatz dazu würde ohne krankhaft gesteigerte Interaktion der Neuronen keine effektive Desynchronisation erfolgen.

Darüber hinaus kann durch die Stimulation mit der Vorrichtung 100 eine Neuorganisation der Konnektivität der gestörten neuronalen Netzwerke erzielt werden, so dass lang anhaltende therapeutische Effekte bewirkt werden können.

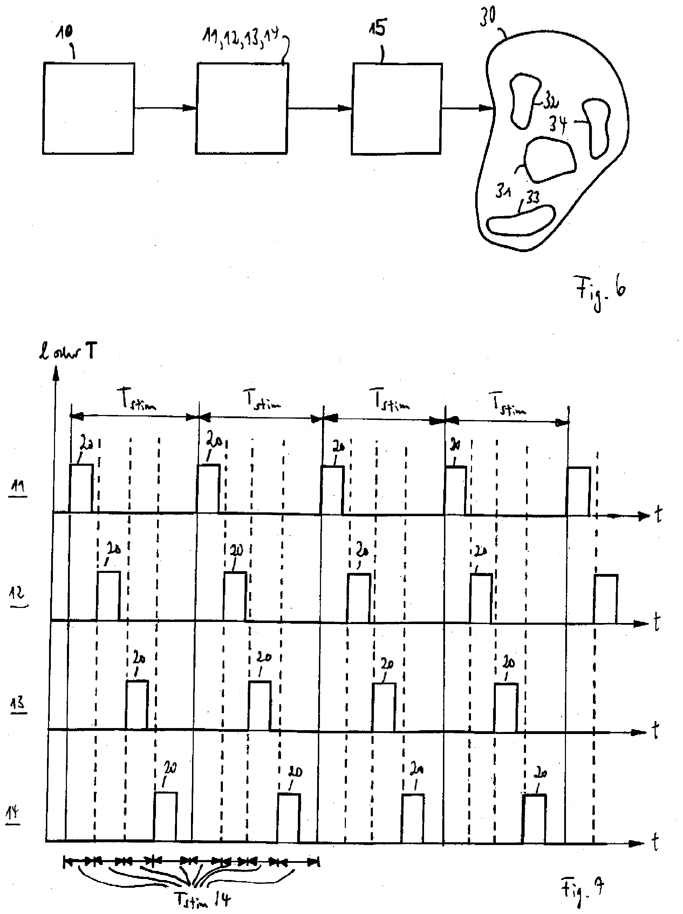

Schematisch ist die Stimulation mehrerer Subpopulationen einer krankhaft aktiven Neuronenpopulation 30 mit Hilfe der Vorrichtung 100 in Fig. 6 dargestellt. Über die Stimulationseinheiten 11 bis 14 werden an unterschiedlichen Stellen der Haut 15 die jeweiligen Rezeptoren mit Vibrations- und/oder Tast- und/oder Thermoreizen 20 stimuliert. Die von den Stimulationseinheiten 11, 12, 13 und 14 applizierten Reize 20 werden an unterschiedliche Subpopulationen 31, 32, 33 bzw. 34 der Neuronenpopulation 30 weitergeleitet (Reize von Stimulationseinheit 11 zu Subpopulation 31, Reize von Stimulationseinheit 12 zu Subpopulation 32, Reize von Stimulationseinheit 13 zu Subpopulation 33 und Reize von Stimulationseinheit 14 zu Subpopulation 34) und resetten die Phasen dieser Subpopulationen zu jeweils unterschiedlichen Zeitpunkten, wodurch eine Desynchronisation der gesamten Neuronenpopulation 30 erzielt wird.

Die gezielte Stimulation bestimmter Bereiche des Gehirns oder Rückenmarks wird durch die somatotope Zuordnung von Körperregionen zu diesen Bereichen ermöglicht. Beispielsweise können die Stimulationseinheiten 11 bis 14 am Fuß, Unterschenkel und Oberschenkel oder aber an der Hand, dem Unteram und Oberam des Patienten angebracht werden. Aufgrund der somatotopischen Gliederung der Nervenleitungsbahnen werden durch die an den jeweiligen Stellen applizierten Reize unterschiedliche Neuronen stimuliert. Die somatotope Zuordnung von Hautstellen zu Bereichen des Gehirns ist beispielsweise in A. Benninghoff et al.: "Lehrbuch der Anatomie des Menschen. Dargestellt unter Bevorzugung funktioneller Zusammenhänge. 3. Bd. Nervensystem, Haut und Sinnesorgane", Urban und Schwarzenberg, München 1964, beschrieben.

Um durch zeitversetztes Zurücksetzen der Phasen der Subpopulationen 31 bis 34 der krankhaft synchronen Neuronenpopulation 30 eine Desynchronisation der gesamten Neuronenpopulation 30 zu erzielen, kann auf verschiedene Art und Weise vorgegangen werden. Beispielsweise können die Reize 20, die ein Zurücksetzen der Phase von Neuronen bewirken, zeitversetzt über die unterschiedlichen Stimulationseinheiten 11 bis 14 an die jeweiligen rezeptiven Felder der Haut abgegeben werden. Des Weiteren können die Reize z.B. phasenversetzt oder mit unterschiedlicher Polarität appliziert werden, so dass sie im Ergebnis auch zu einem zeitversetzten Zurücksetzen der Phasen der unterschiedlichen Subpopulationen 31 bis 34 führen.

Ein für die oben beschriebenen Zwecke geeignetes Stimulationsverfahren ist in Fig. 7 schematisch dargestellt. In Fig. 7 sind untereinander die über die Stimulationseinheiten 11 bis 14 applizierten Reize 20 gegen die Zeit t aufgetragen. Als Reize 20 können beispielsweise die in den Fig. 3A bis 5C dargestellten Vibrations-, Tast- und Thermoreize verwendet werden. Das in Fig. 7 gezeigte Diagramm ist in sich periodisch wiederholende erste Zeitabschnitte der Länge $T_{stim}$ unterteilt. Die Frequenz $f_{stim}$ = $1/T_{stim}$, mit welcher die ersten Zeitabschnitte der Länge $T_{stim}$ wiederholt werden, kann im Bereich von 1 bis 60 Hz und insbesondere im Bereich von 30 bis 60 Hz oder im Bereich von 1 bis 30 Hz oder im Bereich 1 bis 20 Hz oder im Bereich von 5 bis 20 Hz liegen, kann aber auch kleinere oder größere Werte annehmen.

Die ersten Zeitabschnitte der Länge $T_{stim}$ sind ferner in zweite Zeitabschnitte der Länge $T_{stim}/4$ unterteilt. Bei einer Stimulation über N Stimulationseinheiten könnten die ersten Zeitabschnitte in N zweite Zeitabschnitte der Länge $T_{stim}/N$ unterteilt sein.

Gemäß einer Ausgestaltung generiert jede der Stimulationseinheiten 11 bis 14 innerhalb eines ersten Zeitabschnitts nicht mehr als einen Reiz 20. In aufeinander folgenden zweiten Zeitabschnitten können Reize 20 von unterschiedlichen Stimulationseinheiten 11 bis 14 generiert werden.

Bei der in Fig. 7 dargestellten Ausgestaltung appliziert jede der Stimulationseinheiten 11 bis 14 einen Reiz 20 streng periodisch mit der Frequenz $f_{stim}$. Die Verabreichung der Reize 20 über unterschiedliche Stimulationseinheiten 11 bis 14 erfolgt mit einer zeitlichen Verzögerung zwischen den einzelnen Stimulationseinheiten 11 bis 14 um $T_{stim}/4$.

Im Fall von N Stimulationseinheiten kann die zeitliche Verzögerung zwischen jeweils zwei aufeinander folgenden Reizen 20 beispielsweise im Bereich eines N-tels der Periode $1/f_{stim}$ liegen, d.h. $1/(N \times f_{stim}) = T_{stim}/N$, d.h. insbesondere vergeht zwischen den Startzeitpunkten von zwei aufeinander folgenden Reizen 20 die Zeit $T_{stim}/N$.

Die Frequenz $f_{stim}$ kann beispielsweise im Bereich der mittleren Frequenz der krankhaft rhythmischen Aktivität des Ziel-Netzwerks liegen. Bei Erkrankungen, bei denen eine gesteigerte neuronale Synchronisation vorliegt, liegt die mittlere Frequenz typischerweise im Bereich von 1 bis 30 Hz, kann aber auch außerhalb dieses Bereichs liegen. Hierbei ist zu beachten, dass die Frequenz, mit welcher die betroffenen Neuronen synchron feuern, üblicherweise nicht konstant ist, sondern durchaus Variationen aufweisen kann und darüber hinaus bei jedem Patienten individuelle Abweichungen zeigt.

Von dem in Fig. 7 gezeigten streng periodischen Stimulationsmuster kann auf unterschiedliche Art und Weise abgewichen werden. Beispielsweise braucht die zeitliche Verzögerung $T_{stim}$ zwischen aufeinander folgenden und von derselben Stimulationseinheit erzeugten Reize 20 nicht stets gleich groß sein, sondern kann im Bereich von $\pm$ 10% oder $\pm$ 5% oder $\pm$ 3% um $T_{stim}$ herum variieren. Ferner kann auch der Zeitabstand zwischen zwei aufeinander folgenden und von verschiedenen Stimulationseinheiten erzeugten Reize 20 im Bereich von $\pm$ 10% oder $\pm$ 5% oder $\pm$ 3% um $T_{stim}/N$ herum variieren. Es kann durchaus vorgesehen sein, dass die zeitlichen Abstände zwischen den einzelnen Reizen 20 unterschiedlich gewählt werden. Ferner können die Verzögerungszeiten auch während der Behandlung eines Patienten variiert werden. Auch können die Verzögerungszeiten hinsichtlich der physiologischen Signallaufzeiten adjustiert werden.

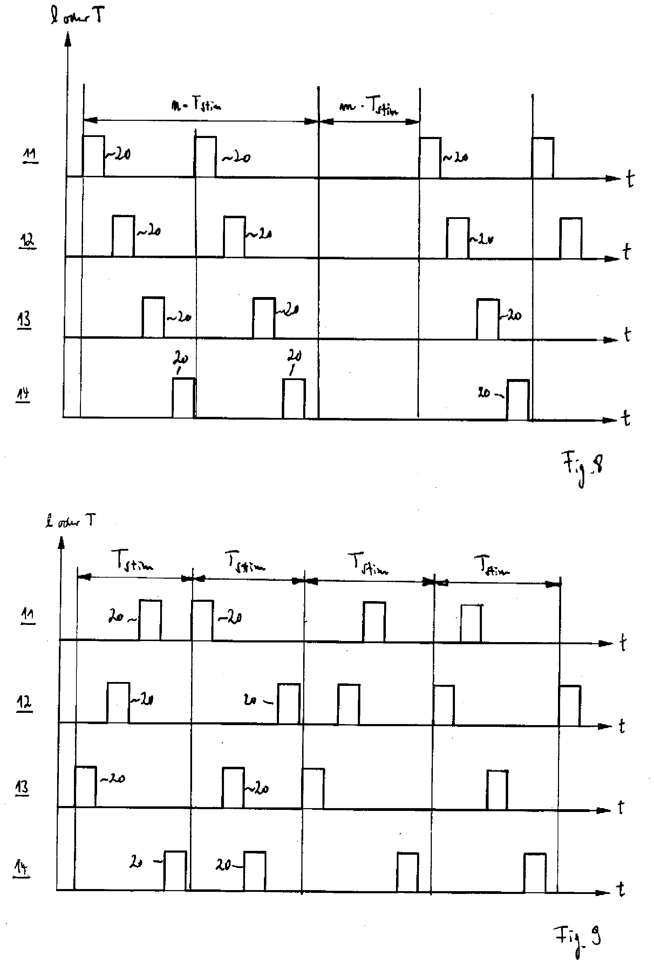

Des Weiteren können während der Applikation der Reize 20 Pausen vorgesehen werden, während derer keine Stimulation erfolgt. Eine solche Pause ist beispielhaft in Fig. 8 gezeigt. Die Pausen können beliebig lang gewählt werden und insbesondere ein ganzzahliges Vielfaches der Periode $T_{stim}$ betragen. Ferner können die Pausen nach einer beliebigen Anzahl von Stimulationen eingehalten werden. Z.B. kann eine Stimulation während n aufeinander folgender Perioden der Länge $T_{stim}$ durchgeführt werden und anschließend eine Pause während m Perioden der Länge $T_{stim}$ ohne Stimulation eingehalten werden, wobei n und m kleine ganze Zahlen sind, z.B. im Bereich von 1 bis 10. Dieses Schema kann entweder periodisch fortgesetzt werden oder stochastisch und/oder deterministisch oder gemischt stochastisch-deterministisch modifiziert werden.

Eine weitere Möglichkeit, von dem in Fig. 7 gezeigten streng periodischen Stimulationsmuster abzuweichen, besteht darin, die zeitliche Abfolge der einzelnen Reize 20 stochastisch oder deterministisch oder gemischt stochastisch-deterministisch zu variieren.

Des Weiteren kann pro Periode $T_{stim}$ (oder auch in anderen Zeitschritten) die Reihenfolge, in welcher die Stimulationseinheiten 11 bis 14 die Reize 20 applizieren, variiert werden, wie dies beispielhaft in Fig. 9 gezeigt ist. Diese Randomisierung kann stochastisch oder deterministisch oder gemischt stochastisch-deterministisch erfolgen.

Die in Fig. 9 gezeigte Randomisierung kann mit der in Fig. 8 gezeigten Stimulationsform kombiniert werden. Beispielsweise kann in jedem der n aufeinander folgenden Stimulationszeitabschnitte der Länge $T_{stim}$ eine erneute Randomisierung durchgeführt werden oder aber es erfolgt nach jeder Pause der Länge m x $T_{stim}$ eine Randomisierung und innerhalb der darauf folgenden n Stimulationszeitabschnitte bleibt die Reihenfolge, in welcher die Stimulationseinheiten 11 bis 14 die Reize 20 applizieren, konstant.

Ferner kann pro Periode $T_{stim}$ (oder in einem anderen Zeitintervall) nur eine bestimmte Anzahl von Stimulationseinheiten 11 bis 14 zur Stimulation herangezogen werden und die an der Stimulation beteiligten Stimulationseinheiten können in jedem Zeitintervall variiert werden. Auch diese Variation kann stochastisch oder deterministisch oder gemischt stochastisch-deterministisch erfolgen.

Es ist denkbar, dass die Stimulation durch den Patienten gestartet wird, beispielsweise durch eine telemetrische Aktivierung. In diesem Fall kann der Patient z.B. mittels eines externen Senders die Stimulation für einen vorgegebenen Zeitraum von z.B. 60 Minuten aktivieren oder der Patient kann die Stimulation selbsttätig starten und beenden.

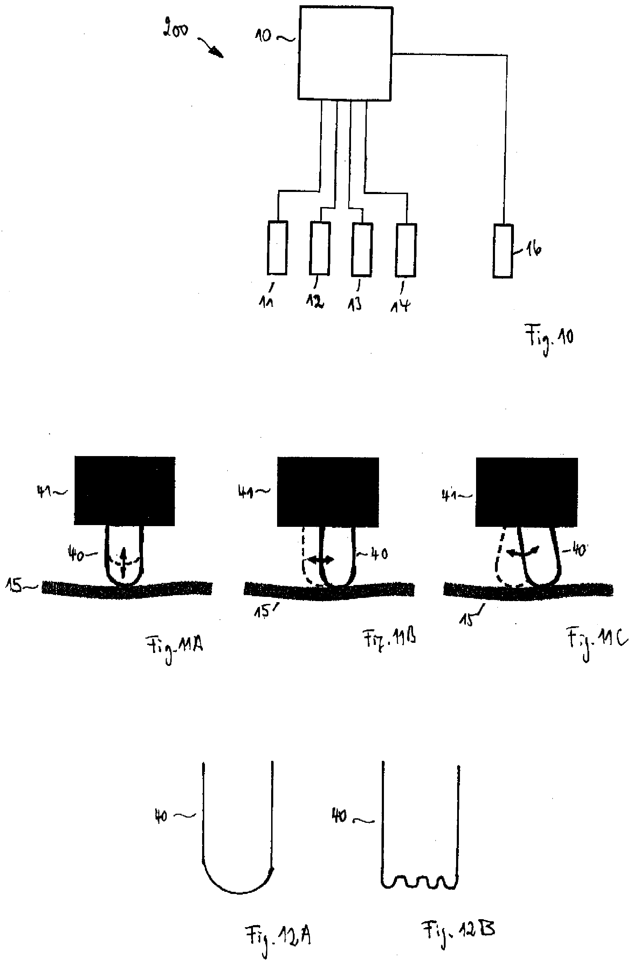

Die Vorrichtung 100 kann beispielsweise in einem sogenannten "open loop"-Modus betrieben werden, bei welchem die Steuereinheit 10 die Stimulationseinheiten 11 bis 14 derart ansteuert, dass diese vorgegebene Reize 20 erzeugen, die an das Hautgewebe abgegeben werden. Des Weiteren kann die Vorrichtung 100 auch zu einer in Fig. 10 gezeigten Vorrichtung 200 weitergebildet werden, welche ein sogenanntes "closed loop"-System darstellt. Die Vorrichtung 200 enthält zusätzlich noch eine Messeinheit 16, welche ein oder mehrere am Patienten aufgenommene Messsignale bereitstellt und diese an die Steuereinheit 10 weiterleitet. Es kann vorgesehen sein, dass die Steuereinheit 10 anhand der von der Messeinheit 16 aufgenommenen Messsignale die Stimulationseinheiten 11 bis 14 ansteuert.

Bei der Messeinheit 16 kann es sich um nicht-invasive Sensoren handeln, wie z.B. Elektroenzephalographie (EEG)-Elektroden, Magnetenzephalographie (MEG)-Sensoren, Akzelerometer, Elektromyographie (EMG)-Elektroden und Sensoren zur Bestimmung von Blutdruck, Atmung oder Hautleitwiderstand. Ferner kann die Messeinheit 16 in Form eines oder mehrerer Sensoren in den Körper des Patienten implantiert sein. Als invasive Sensoren können beispielsweise Tiefenhirnelektroden, sub- oder epidurale Hirnelektroden, subkutane EEG-Elektroden und sub- oder epidurale Rückenmarkselektroden dienen. Des Weiteren können an peripheren Nerven zu befestigende Elektroden als Sensoren eingesetzt werden. Insbesondere kann mittels der Messeinheit 16 die neuronale Aktivität in dem stimulierten Zielgebiet, d.h. z.B. die neuronale Aktivität der in Fig. 6 schematisch dargestellten Neuronenpopulation 30, oder einem damit verbundenen Gebiet gemessen werden.

Hinsichtlich des Zusammenwirkens der Steuereinheit 10 mit der Messeinheit 16 sind verschiedene Ausgestaltungen denkbar. Beispielsweise kann von der Steuereinheit 10 eine bedarfsgesteuerte Stimulation durchgeführt werden. Hierzu detektiert die Steuereinheit 10 anhand der von der Messeinheit 16 aufgenommenen Messsignale das Vorhandensein und/oder die Ausprägung eines oder mehrerer krankhafter Merkmale. Beispielsweise kann die Amplitude oder der Betrag der neuronalen Aktivität gemessen werden und mit einem vorgegebenen Schwellwert verglichen werden. Die Steuereinheit 10 kann so ausgestaltet sein, dass eine Stimulation gestartet wird, sobald der vorgegebene Schwellwert überschritten wird. Alternativ zum Steuern der Zeitpunkte der Stimulation anhand der von der Messeinheit 16 aufgenommenen Messsignale oder zusätzlich dazu kann von der Steuereinheit 10 anhand der Ausprägung der krankhaften Merkmale beispielsweise die Stärke der Reize eingestellt werden. Z.B. können ein oder mehrere Schwellwerte vorgeben werden, und bei einem Überschreiten der Amplitude oder des Betrags der Messsignale über einen bestimmten Schwellwert stellt die Steuereinheit 10 eine bestimmte Stärke der Reize 20, z.B. eine bestimmte Frequenz $f_{vib}$ oder Eindrücktiefe $I_2$ im Fall von Vibrationsreizen, ein.

Des Weiteren kann vorgesehen sein, dass die von der Messeinheit 16 aufgenommenen Messsignale direkt oder gegebenenfalls nach einem oder mehreren Verarbeitungsschritten als Reize 20 verwendet werden und von der Steuereinheit 1 in eine oder mehrere der Stimulationseinheiten 11 bis 14 eingespeist werden. Beispielsweise können die Messsignale verstärkt und gegebenenfalls nach mathematischer Verrechnung (z.B. nach Mischung der Messsignale) mit einer Zeitverzögerung und linearen und/oder nichtlinearen Verrechnungsschritten und Kombinationen prozessiert und in mindestens eine der Stimulationseinheiten 11 bis 14 eingespeist werden. Der Verrechnungsmodus wird hierbei so gewählt, dass der krankhaften neuronalen Aktivität entgegengewirkt wird und das Stimulationssignal mit abnehmender krankhafter neuronaler Aktivität ebenfalls verschwindet oder zumindest deutlich in seiner Stärke reduziert wird. Beispielsweise können die (evtl. weiterverarbeiteten) Messsignale analog zum Stimulationsverfahren nach Fig. 7 mit einer zeitlichen Verzögerung von $T_{stim}/4$ über die einzelnen Stimulationseinheiten 11 bis 14 appliziert werden.

Diese Art der Stimulation, bei welcher die am Patienten aufgenommenen Messsignale zur Desynchronisation einer Neuronenpopulation wieder in den Körper des Patienten eingespeist werden, könnte grundsätzlich auch mit nur einer einzigen Stimulationseinheit durchgeführt werden, es kann jedoch auch eine beliebige, größere Anzahl von Stimulationseinheiten vorgesehen sein.

Zur Erzeugung der Reize 20 können die Messsignale beispielsweise verstärkt und gegebenenfalls nach mathematischer Verrechnung (z.B. nach Mischung der Messsignale) mit einer Zeitverzögerung und linearen und/oder nichtlinearen Verrechnungsschritten zur elektrischen Ansteuerung der Stimulationseinheiten verwendet werden, welche die Messsignale dann in Vibrations-, Tast- bzw. Thermoreize umsetzen. Der Verrechnungsmodus kann hierbei so gewählt werden, dass der krankhaften neuronalen Aktivität entgegengewirkt wird und der applizierte Reiz mit abnehmender krankhafter neuronaler Aktivität ebenfalls verschwindet oder zumindest deutlich in seiner Stärke reduziert wird.

Im Folgenden werden lineare und nicht-lineare Verarbeitungsschritte beschrieben, mit denen die mit Hilfe der Messeinheit 16 gewonnenen Messsignale prozessiert werden können, bevor sie zur Ansteuerung der Stimulationseinheiten verwendet werden. Bei einer nicht-linearen Verarbeitung der Messsignale wird nicht die Phase der neuronalen Aktivität in den jeweiligen stimulierten Subpopulationen zurückgesetzt, sondern die Synchronisation in der krankhaft aktiven Neuronenpopulation wird unterdrückt, indem der Sättigungsprozess der Synchronisation beeinflusst wird.

Bei einer linearen Verarbeitung eines von der Messeinheit 16 gewonnenen Messsignals kann das Messsignal beispielsweise gefiltert und/oder verstärkt und/oder mit einer Zeitverzögerung beaufschlagt werden, bevor das so verarbeitete Signal in die Stimulationseinheit eingespeist wird und in einen Vibrations- und/oder Tast- und/oder Thermoreiz umgesetzt wird. Als Beispiel sei angenommen, dass das Messsignal mittels einer EEG-Elektrode aufgenommen worden sei und die pathologische Aktivität im Zielgebiet wiedergebe. Dementsprechend ist das Messsignal eine Sinusschwingung mit einer Frequenz im Bereich von 1 bis 30 Hz. Weiterhin sei beispielhaft angenommen, dass das Messsignal eine Frequenz von 5 Hz aufweise. Das Messsignal kann mittels eines Bandpassfilters mit einem Durchlassbereich im Bereich von 5 Hz gefiltert werden und mittels eines Verstärkers so verstärkt werden, dass es einen für die Ansteuerung der Stimulationseinheit geeigneten Pegel aufweist. Anschließend wird die so erhaltene verstärkte Sinusschwingung zur Ansteuerung der Stimulationseinheit verwendet. Im Falle einer Stimulationseinheit zur Applikation von Vibrations- oder Tastreizen führt das Stimulationselement dann eine sinusförmige Bewegung mit einer Frequenz von 5 Hz aus.

Sofern zur Stimulation eine Anzahl N von Stimulationseinheiten herangezogen wird, kann das Messsignal mit den in Fig. 7 gezeigten zeitlichen Verzögerungen $T_{stim}/N$ beaufschlagt werden, bevor es in die entsprechenden Stimulationseinheiten eingespeist wird.

Im Folgenden wird anhand eines Beispiels erläutert, wie ein von der Messeinheit 16 gewonnenes Messsignal einer nichtlinearen Prozessierung unterworfen werden kann, bevor es als Stimulationsreiz verwendet wird. Genauso wie bei der linearen Verarbeitung kann das Messsignal auch hier gefiltert und/oder verstärkt und/oder mit einer Zeitverzögerung beaufschlagt werden.

Ausgangspunkt ist eine Gleichung für den Stimulationsreiz S (t) :

$$S(t) = K \cdot \overline{Z}^2(t) \cdot \overline{Z}^*(t - \tau) \qquad (2)$$

In Gleichung (2) sind K ein Verstärkungsfaktor, der geeignet gewählt werden kann, und $\overline{Z}(t)$ eine mittlere Zustandsvariable des Messsignals. $\overline{Z}(t)$ ist eine komplexe Variable und kann folgendermaßen dargestellt werden:

$$\overline{Z}(t) = X(t) + iY(t), \qquad (3)$$

wobei X(t) z.B. dem neurologischen Messsignal entsprechen kann, und i die imaginäre Einheit ist. Da die betrachteten Frequenzen im Bereich von 10 Hz = 1/100ms = $1/T\alpha$ liegen, kann der Imaginärteil Y(t) durch $X(t - \tau\alpha)$ angenähert werden, wobei beispielsweise $\tau\alpha = T\alpha/4$ gilt. Damit ergibt sich:

$$S(t) = K \cdot [X(t) + iX(t - \tau_\alpha)]^2 \cdot [X(t - \tau) - iX(t - \tau - \tau_\alpha)] \qquad (4)$$

Gleichung (4) kann folgendermaßen umgeformt werden:

$$\begin{aligned}
S(t) = K \cdot \big[ &X(t)^2 \cdot X(t - \tau) + i2X(t) \cdot X(t - \tau_\alpha) \cdot X(t - \tau) - X(t - \tau_\alpha) \cdot X(t - \tau) \\
&- iX(t - \tau - \tau_\alpha) \cdot X(t)^2 + 2X(t) \cdot X(t - \tau_\alpha) \cdot X(t - \tau - \tau_\alpha) \\
&+ iX(t - \tau - \tau_\alpha) \cdot X(t - \tau_\alpha) \big] \qquad (5)
\end{aligned}$$

Als Stimulationsreiz wird der Realteil aus Gleichung (5) verwendet:

$$\mathrm{real}[S(t)] = K \cdot \big[ X(t)^2 \cdot X(t - \tau) - X(t - \tau_\alpha) \cdot X(t - \tau) + 2X(t) \cdot X(t - \tau_\alpha) \cdot X(t - \tau - \tau_\alpha) \big] \qquad (6)$$

Fig. 11A bis 11C zeigen schematisch verschiedene Möglichkeiten zur Realisierung einer Stimulationseinheit zur Erzeugung von Vibrations- und/oder Tastreizen, wie sie in den Fig. 3A bis 4 gezeigt sind. Die Stimulationseinheiten umfassen ein Stimulationselement 40, beispielsweise in Form eines Stabs, mit dessen einem Ende die Haut 15 des Patienten stimuliert wird. Angetrieben wird das Stimulationselement 40 von einem elektromechanischen Wandler 41 (oder Aktor oder Aktuator), der elektrische Energie in eine Bewegung des Stimulationselements 40 umsetzt. Als elektromechanische Wandler 41 eignen sich beispielsweise Gleichstrommotoren, Schwingspulen (engl.: voice coil), piezoelektrische Wandler oder aus elektroaktiven Polymeren (EAP) aufgebaute Wandler, die beim Anlegen einer elektrischen Spannung ihre Form ändern.

Die elektromechanischen Wandler 41 können so ausgelegt sein, dass das Stimulationselement 40 senkrecht zur Hautoberfläche ausgelenkt wird (vgl. Fig. 11A) oder parallel dazu (vgl. Fig. 11B). Die Bewegung des Stimulationselements 40 kann aber auch auf beliebigen anderen Bahnen erfolgen. Als Beispiel dafür ist in Fig. 11C eine pendelförmige Auslenkung des Stimulationselements 40 dargestellt.

Das Ende des Stimulationselements 40, das in Berührung mit der Hautoberfläche kommt und letztlich die Reize erzeugt, kann beispielsweise im Wesentlichen die Form einer Halbkugel aufweisen (vgl. Fig. 12A) oder eine noppenartige Oberfläche haben (vgl. Fig. 12B) oder eine andere geeignete Form haben.

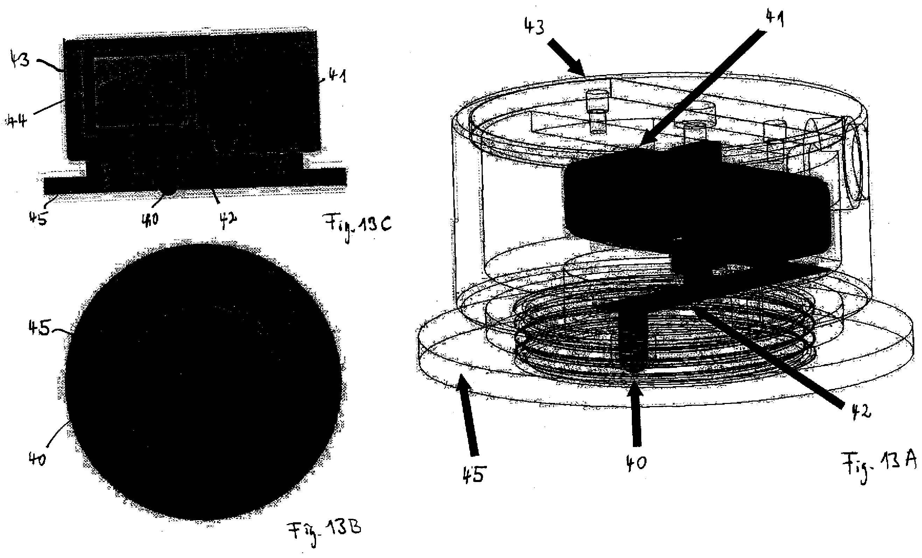

In den Fig. 13A bis 13C ist eine Ausgestaltung eines Stimulationselements zur Applikation von Vibrations- und/oder Tastreizen in Durchsicht (vgl. Fig. 13A), Draufsicht von unten (vgl. Fig. 13B) und im Querschnitt (vgl. Fig. 13C) dargestellt. Das vorliegende Stimulationselement enthält einen Piezoaktuator 41 als elektromechanischen Wandler. Da die Auslenkung des Piezoaktuators 41 für die beabsichtigten Zwecke nicht ausreichend ist, kann ein Mechanismus zur Verstärkung der Auslenkung des Piezoaktuators 41 vorgesehen sein. Beispielhaft ist hier ein Hebelarm 42 gezeigt, der die Bewegung des Piezoaktuators 41 verstärkt. Der Hebelarm ist vorliegend eine längliche Biegefeder 42, die mit ihrem einen Ende am Gehäuse 43 des Stimulationselements befestigt ist und an deren anderem Ende das Stimulationselement 40 angebracht ist. Der Piezoaktuator 41 drückt auf die Oberseite der Biegefeder 42 und das an der Unterseite der Biegefeder 42 angebrachte Stimulationselement 40 folgt der Auslenkung des Piezoaktuators 41 mit einer aufgrund der geometrischen Anordnung verstärkten Amplitude und appliziert die Vibrations- und/oder Tastreize auf die Haut des Patienten. Die Unterseite des Stimulationselements 40, die mit der Haut in Berührung kommt, kann verschiedene Geometrien und Abmessungen aufweisen. Beispielsweise kann das Stimulationselement 40 an seiner Unterseite flach, rund oder ungleichförmig sein.

In dem Gehäuse 43 des Stimulationselements, das den Piezoaktuator 41 und den Verstärkungsmechanismus beherbergt, kann ferner ein Raum 44 für Elektronik und Verbindungsanschlüsse vorgesehen sein. Außerdem ist an der Unterseite des Gehäuses 43 ein Verstellring 45 angebracht, der mit dem Gehäuse 43 über ein Gewinde verbunden ist und der eine Verstellung der Höhe ermöglicht, um die das Stimulationselement 40 in seiner Ruheposition von der Unterseite der Stimulationseinheit hervorsteht. Während des Betriebs sitzt die Stimulationseinheit mit ihrer Unterseite auf der Haut des Patienten und ist beispielsweise mit einer geeigneten Manschette am Körper des Patienten befestigt. Zusätzlich oder alternativ zu der Manschette könnte die Stimulationseinheit noch mit einem ein- oder doppelseitigen medizinischen Klebeband an der Haut des Patienten befestigt sein. Das Gehäuse 43 schützt den Patienten vor möglichen Gefahren, wie z.B. elektrischer Spannung.

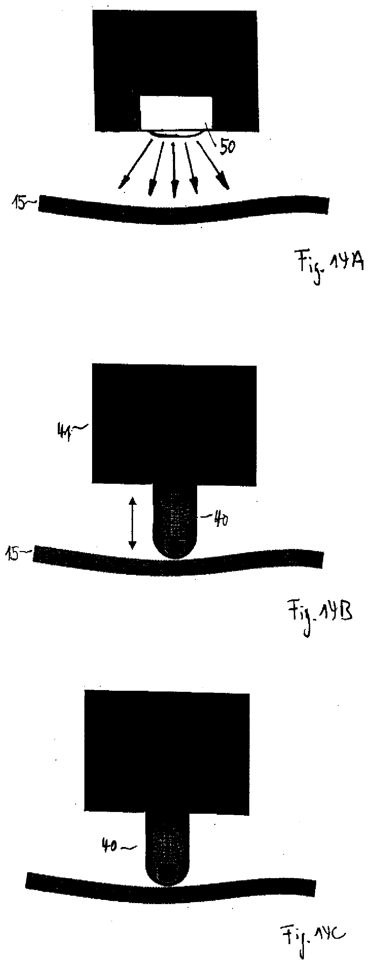

Fig. 14A bis 14B zeigen schematisch verschieden ausgestaltete Stimulationseinheiten zur Erzeugung von Thermoreizen, wie sie in den Fig. 5A bis 5C dargestellt sind. Die in Fig. 14A dargestellte Stimulationseinheit arbeitet kontaktlos und bewirkt durch das Licht einer Infrarot-LED 50 eine Erwärmung der Haut.

Stimulationseinheiten, die durch Berührung der Hautoberfläche Thermoreize applizieren, sind in den Fig. 14B und 14C gezeigt. Die in Fig. 14B gezeigte Stimulationseinheit enthält mit einem elektromechanischen Wandler 41 und einem stabförmigen Stimulationselement 40 im Wesentlichen die gleichen Bauelemente wie die Stimulationseinheit aus Fig. 11A. Zusätzlich weist die Stimulationseinheit aus Fig. 14B ein Heiz- und/oder Kühlelement auf (z.B. in Form einer Heizschleife), welches das Stimulationselement heizt oder kühlt. Die Thermoreize werden durch die Bewegungen des Stimulationselements 40 erzeugt, bei welchen das Stimulationselement 40 wiederholt in Kontakt mit der Haut 15 kommt und wieder entfernt wird. Die Temperatur des Stimulationselements 40 kann während der gesamten Stimulation konstant sein.

Alternativ kann das beheizbare bzw. kühlbare Stimulationselement 40 wie in Fig. 14C gezeigt während des gesamten Stimulationszeitraums in Kontakt mit der Haut 15 des Patienten stehen. Die Thermoreize werden in diesem Fall durch eine zeitliche Variation der Temperatur des Stimulationselements 40 generiert. Ein elektromechanischer Wandler ist bei dieser Ausgestaltung nicht zwingend erforderlich.

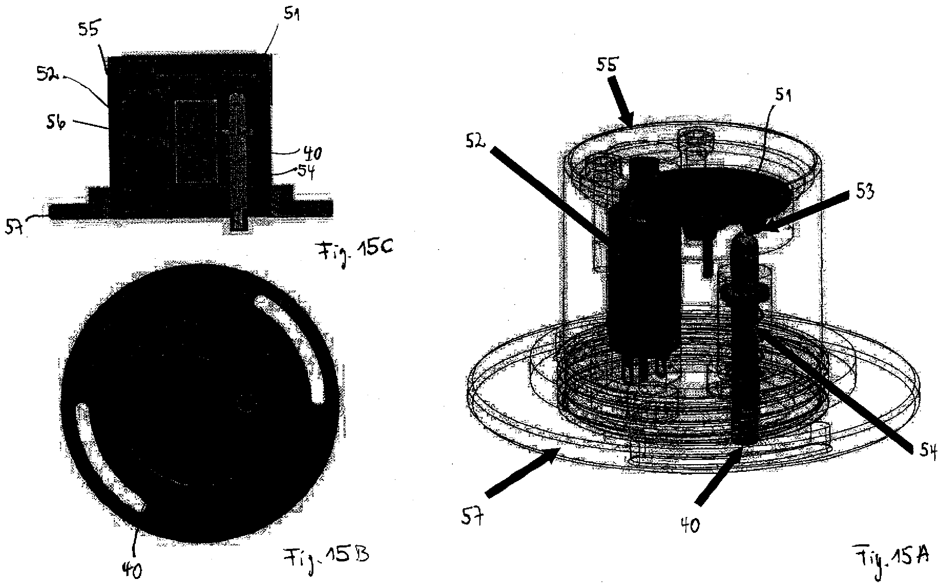

In den Fig. 15A bis 15C ist eine Ausgestaltung eines Stimulationselements zur Applikation von Thermoreizen in Durchsicht (vgl. Fig. 15A), Draufsicht von unten (vgl. Fig. 15B) und im Querschnitt (vgl. Fig. 15C) dargestellt. Die Stimulationseinheit enthält ein stabförmiges Stimulationselement 40, dessen unteres Ende beheizbar und/oder kühlbar ist. An seinem oberen Ende wird das Stimulationselement 40 von einer Nockenscheibe 51 angetrieben. Während der Stimulation versetzt ein Gleichstrommotor 52 die Nockenscheibe 51 in Rotation. Durch die an der Unterseite der Nockenscheibe 51 angebrachten Nocken 53 wird das Stimulationselement 40 nach unten hin ausgelenkt. Eine Rückstellfeder 54 sorgt dafür, dass das Stimulationselement 40 anschließend wieder in seine Ausgangslage zurückkehrt. Durch diesen Mechanismus wird die Rotationsbewegung der Nockenscheibe 51 in eine lineare Bewegung des Stimulationselements 40 umgewandelt. Wie oben beschrieben kann das Stimulationselement 40 entweder für eine gewisse Zeit mit der Haut des Patienten in Kontakt stehen oder aber das Stimulationselement 40 wird durch eine Rotation der Nockenscheibe 51 zyklisch auf die Haut gebracht und wieder entfernt.

Die Bauteile der Stimulationseinheit können in ein Gehäuse 55 eingebracht sein. In dem Gehäuse 55 kann ein Raum 56 für Elektronik und Verbindungsanschlüsse vorgesehen sein. Außerdem kann an der Unterseite des Gehäuses 55 ein Verstellring 57 angebracht sein, der mit dem Gehäuse 55 über ein Gewinde verbunden ist und der eine Verstellung der Höhe ermöglicht, um die das Stimulationselement 40 in seiner Ruheposition von der Unterseite der Stimulationseinheit hervorsteht (das Stimulationselement 40 kann aufgrund des Verstellrings in seiner Ruheposition auch vollständig oberhalb der Unterseite des Verstellrings liegen). Während des Betriebs sitzt die Stimulationseinheit mit ihrer Unterseite auf der Haut des Patienten und ist beispielsweise mit einer geeigneten Manschette am Körper des Patienten befestigt. Zusätzlich oder alternativ zu der Manschette könnte die Stimulationseinheit noch mit einem ein- oder doppelseitigen medizinischen Klebeband an der Haut des Patienten befestigt sein. Das Gehäuse 55 schützt den Patienten vor möglichen Gefahren, wie z.B. elektrischer Spannung.

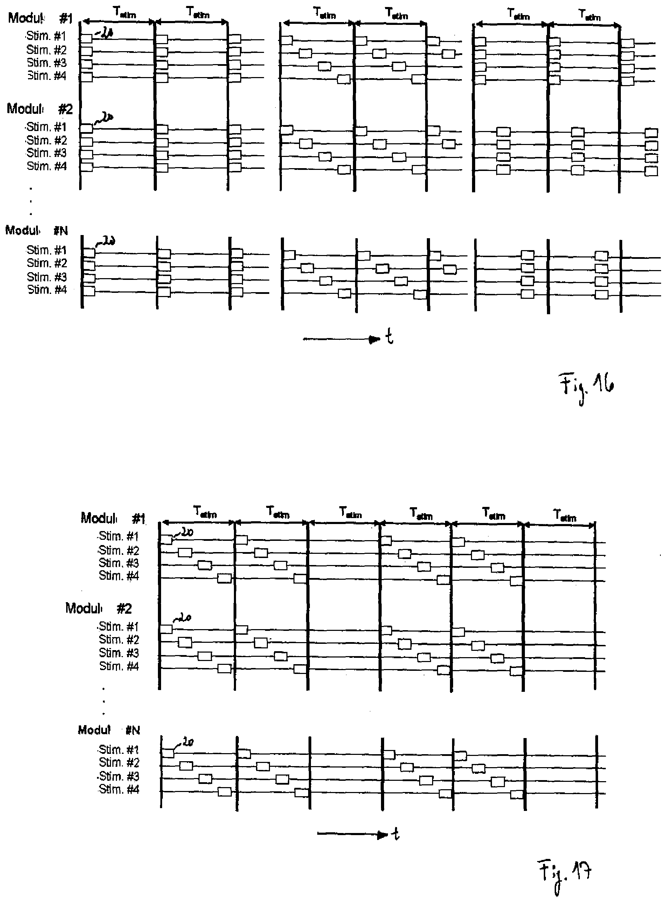

Die in dieser Anmeldung beschriebenen Stimulationseinheiten können einzeln am Patienten befestigt werden oder können auch zu mehreren in ein Modul integriert werden. Beispielsweise kann ein Modul eine Manschette mit mehreren daran befestigten Stimulationseinheiten umfassen. Die Manschette kann dann an einem Arm oder Bein des Patienten befestigt werden. Fig. 16 zeigt Stimulationsverfahren, wie sie mit insgesamt N Modulen, die jeweils z.B. vier Stimulationseinheiten enthalten, durchgeführt werden können. Bei dem in Fig. 16 ganz links dargestellten Stimulationsverfahren applizieren alle Stimulationseinheiten zu Beginn einer Stimulationsperiode $T_{stim}$ einen Vibrations-, Tast- oder Thermoreiz 20. Bei dem in der Mitte von Fig. 16 gezeigten Stimulationsverfahren sind die Reize 20 der vier verschiedenen Stimulationseinheiten eines Moduls jeweils um $T_{stim}/4$ gegeneinander verschoben. In diesem Fall appliziert in jedem Zeitabschnitt der Länge $T_{stim}/4$ genau eine Stimulationseinheit jedes Moduls einen Reiz 20. Bei dem in Fig. 16 ganz rechts dargestellten Stimulationsverfahren erzeugen die vier Stimulationseinheiten eines Moduls ihre Reize 20 gleichzeitig, jedoch sind die Reize 20 unterschiedlicher Module gegeneinander verschoben.

Bei allen in Fig. 16 gezeigten Stimulationsverfahren können auch beliebige Pausen während der Stimulation eingehalten werden. Typischerweise haben die Stimulationspausen die Länge einer oder mehrerer Stimulationsperioden $T_{stim}$. Beispielhaft ist dies in Fig. 17 gezeigt. Bei dem dort dargestellten Stimulationsverfahren wird eine Stimulation während zwei aufeinander folgender Stimulationsperioden $T_{stim}$ durchgeführt, danach wird während einer Stimulationsperiode $T_{stim}$ eine Stimulationspause eingehalten. Dieses Muster wiederholt sich periodisch.

Des Weiteren kann den in den Fig. 16 und 17 gezeigten Stimulationsverfahren eine Randomisierung der Reihenfolge, in welcher die einzelnen Stimulationseinheiten Reize generieren, hinzugefügt werden, wobei u.a. folgende Randomisierungen denkbar sind:

1. Randomisierung der Reizsequenzen für jede Stimulationsperiode $T_{stim}$ kohärent über alle Module, d.h. zu Beginn jeder Stimulationsperiode $T_{stim}$ wird eine Reihenfolge festgelegt, in der die Stimulationseinheiten die Reize generieren (z.B. die Reihenfolge Stim. #4, Stim. #2, Stim. #3, Stim. #1) und diese Reihenfolge gilt für alle Module.

2. Randomisierung der Reizsequenzen für einen Block von aufeinander folgenden Stimulationsperioden $T_{stim}$ kohärent über alle Module, d.h. zu Beginn eines in Fig. 17 gezeigten Blocks von aufeinander folgenden Stimulationsperioden $T_{stim}$ (bzw. nach einer Stimulationspause) wird eine Reihenfolge festgelegt, in der die Stimulationseinheiten die Reize generieren (z.B. die Reihenfolge Stim. #4, Stim. #2, Stim. #3, Stim. #1) und diese Reihenfolge gilt für alle Module für den Stimulationsblock bis zur nächsten Pause.

3. Randomisierung der Reizsequenzen nicht kohärent über alle Module, sondern nur kohärent über eine Untergruppe aller Module variiert, d.h. nur für ein bestimmtes Modul (z.B. das Modul #2) wird eine Randomisierung gemäß den vorstehenden Ziffern 1. oder 2. durchgeführt, die übrigen Module verhalten sich wie in Fig. 16 gezeigt.

4. Randomisierung der Reizsequenzen nicht kohärent über alle Module, sondern kohärent über mehr als eine Untergruppe aller Module variiert, d.h. nur für zwei oder mehr Module (z.B. die Module #2 und #4) wird eine Randomisierung gemäß den vorstehenden Ziffern 1. oder 2. durchgeführt, die übrigen Module verhalten sich wie in Fig. 16 gezeigt.

5. Randomisierung der Reizsequenzen unkorreliert zwischen verschiedenen Modulen, d.h. für jede Stimulationsperiode $T_{stim}$ oder für jeden Block von aufeinander folgenden Stimulationsperioden $T_{stim}$ zwischen zwei Pausen wird für jedes Modul unabhängig von den anderen Modulen eine Reihenfolge, in der die Stimulationseinheiten die Reize generieren, festgelegt.

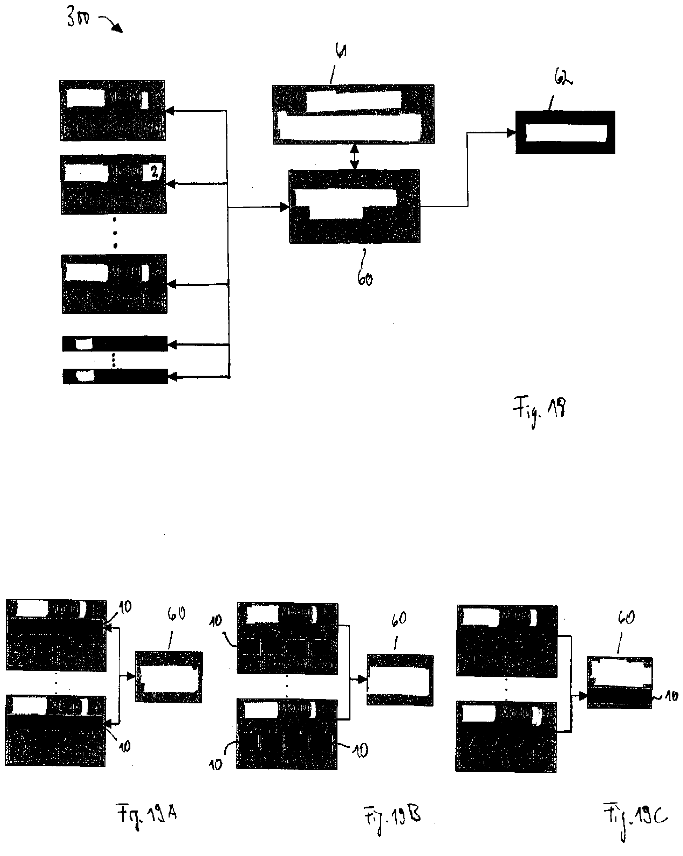

In Fig. 18 ist schematisch das Blockschaltbild einer Vorrichtung 300 zur Erzeugung von Vibrations- und/oder Tast- und/oder Thermoreizen dargestellt. Die Vorrichtung 300 enthält n Module mit jeweils n Stimulationseinheiten sowie n Sensoren. Die Module und Sensoren stehen über Verbindungsleitungen oder über Funk (z.B. ein WPAN (Wireless Personal Area Network)-Netzwerk) mit einem Verbindungsmodul 60 in Verbindung, welches wiederum an einen Computer 61, z.B. ein Laptop, und externe Vorrichtungen 62 angeschlossen sein kann. Es müssen nicht notwendigerweise alle Module und Sensoren gleichzeitig zum Einsatz kommen, es kann je nach Stimulationsart auch nur eine Teilmenge davon eingesetzt werden. Die Module und/oder Sensoren können durch Batterien oder Akkus mit Strom versorgt werden, so dass sie unabhängig von einer zentralen Stromversorgung sind. Der Benutzer, beispielsweise ein Arzt, kann mittels einer geeigneten, auf dem Computer 61 abgespeicherten Software ein Stimulationsverfahren auswählen und die Parameter dieses Stimulationsverfahrens einstellen.

Die Steuerung der in die Module integrierten Stimulationseinheiten kann über den Computer 61 erfolgen. Als Alternative kann in jedes Modul eine Steuereinheit 10 integriert sein (vgl. Fig. 19A), die für die Ansteuerung der Stimulationseinheiten des jeweiligen Moduls zuständig ist. Dies ermöglicht einen weitgehend eigenständigen Betrieb der Module. Ferner kann für jede Stimulationseinheit eine eigene Steuereinheit 10 vorgesehen sein (vgl. Fig. 19B). Dies ermöglicht die größte Vielseitigkeit beim Betrieb der Stimulationseinheiten, jedoch werden dadurch Gewicht und Abmessungen der Module vergrößert. Als weitere Alternative kann die Steuereinheit 10 zentral in dem Verbindungsmodul 60 platziert werden. Vorteilhaft daran sind geringes Gewicht und Größe der Module sowie eine kostengünstige Herstellung. Allerdings können bei dieser Ausgestaltung die Module nicht unabhängig von dem Verbindungsmodul 60 betrieben werden.

## Patentansprüche

1.  Vorrichtung (100 - 300) zur Behandlung eines Patienten mit Vibrations- und/oder Tast- und/oder Thermoreizen (20), umfassend:

    eine erste Stimulationseinheit (11) zur Verabreichung von ersten Reizen (20) an die Haut des Patienten, und eine zweite Stimulationseinheit (12) zur Verabreichung von zweiten Reizen (20) an die Haut des Patienten, wobei die ersten und zweiten Reize (20) jeweils Vibrations- und/oder Tast- und/oder Thermoreize sind,
    die erste Stimulationseinheit (11) derart ausgestaltet ist, dass sie die ersten Reize (20) erzeugt, die bei einer Aufnahme über in der Haut und/oder den Muskeln und/oder dem Subkutangewebe und/oder den Sehnen des Patienten gelegene Rezeptoren an eine erste Subpopulation einer Population von Neuronen, die eine krankhaft synchrone und oszillatorische Aktivität aufweisen, weitergeleitet werden und die ersten Reize (20) bewirken, dass die Phase der neuronalen Aktivität der ersten Subpopulation zurückgesetzt wird,
    die zweite Stimulationseinheit (12) derart ausgestaltet ist, dass sie die zweiten Reize (20) erzeugt, die bei einer Aufnahme über in der Haut und/oder den Muskeln und/oder dem Subkutangewebe und/oder den Sehnen des Patienten gelegene Rezeptoren an eine zweite Subpopulation der Population von Neuronen, die die krankhaft synchrone und oszillatorische Aktivität aufweisen, weitergeleitet werden und die zweiten Reize (20) bewirken, dass die Phase der neuronalen Aktivität der zweiten Subpopulation zurückgesetzt wird, und
    die erste und zweite Stimulationseinheit (11, 12) derart ausgestaltet sind, dass die ersten und zweiten Reize (20) zu zumindest teilweise unterschiedlichen Zeitpunkten verabreicht werden, so dass die Phasen der neuronalen Aktivität der ersten und zweiten Subpopulation zu unterschiedlichen Zeitpunkten zurückgesetzt werden und die erste und zweite Subpopulation nach den durch die ersten und zweiten Reize (20) bewirkten Phasenrücksetzungen unterschiedliche Phasen ihrer neuronalen Aktivität aufweisen.

2.  Vorrichtung(100 - 300) nach Anspruch 1, wobei die erste und zweite Stimulationseinheit (11, 12) derart ausgestaltet sind, dass die ersten und zweiten Reize (20) während eines Stimulationszeitraums jeweils im Mittel mit einer Frequenz $f_{stim}$ von 1 bis 60 Hz wiederholt werden.

## Claims

1.  An apparatus (100 - 300) for treating a patient using vibration stimuli (20) and/or tactile stimuli (20) and/or thermal stimuli (20), comprising:

    a first stimulation unit (11) for administering first stimuli (20) to the skin of the patient, and a second stimulation unit (12) for administering second stimuli (20) to the skin of the patient, wherein
    the first and the second stimuli (20) are vibration stimuli and/or tactile stimuli and/or thermal stimuli, respectively,
    the first stimulation unit (11) is configured for generating the first stimuli (20), which, upon being received by receptors of the patient disposed in the skin and/or the muscles and/or the sub-cutaneous tissue and/or the sinews of the patient, are forwarded to a first subpopulation of a population of neurons having a pathologically synchronous and oscillatory activity, and the first stimuli (20) effect that the phase of the neuronal activity of the first subpopulation is reset,

the second stimulation unit (12) is configured for generating the second stimuli (20), which, upon being received by receptors of the patient disposed in the skin and/or the muscles and/or the sub-cutaneous tissue and/or the sinews of the patient, are forwarded to a second subpopulation of the population of neurons having the pathologically synchronous and oscillatory activity, and the second stimuli (20) effect that the phase of the neuronal activity of the second subpopulation is reset, and

the first and the second stimulation unit (11, 12) are configured for administering the first and second stimuli (20) at least partly at different times such that the phases of the neuronal activity of the first and the second subpopulation are reset at different times and, after the phase resets effected by the first and second stimuli (20), the phases of the neuronal activity of the first and the second subpopulation is different.

2. Apparatus (100 - 300) according to claim 1, wherein the first and the second stimulation unit (11, 12) are configured such that each of the first and the second stimuli (20) are repeated on average at a frequency $f_{stim}$ of 1 to 60 Hz during a stimulation period.

## Revendications

1. Dispositif (100 - 300) de traitement d'un patient par des stimuli vibratoires et/ou tactiles et/ou thermiques (20), comprenant :

une première unité de stimulation (11) pour administrer des premiers stimuli (20) à la peau du patient, et une seconde unité de stimulation (12) pour administrer des seconds stimuli (20) à la peau du patient, dans lequel les premier et les second stimuli (20) sont respectivement des stimuli vibratoires et/ou tactiles et/ou thermiques, la première unité de stimulation (11) est conçue de manière à générer les premiers stimuli (20) qui, lorsqu'ils sont reçus, sont réacheminés par l'intermédiaire de récepteurs situés dans la peau et/ou les muscles et/ou le tissu sous-cutané et/ou les tendons du patient vers une première sous-population d'une population de neurones présentant une activité pathologiquement synchrone et oscillatoire, et les premiers stimuli (20) provoquent la réinitialisation de la phase de l'activité neuronale de la première sous-population, la seconde unité de stimulation (12) est conçue de manière à générer les seconds stimuli (20) qui, lorsqu'ils sont reçus, sont réacheminés par l'intermédiaire de récepteurs situés dans la peau et/ou les muscles et/ou le tissu sous-cutané et /ou les tendons du patient vers une seconde sous-population de la population de neurones présentant l'activité pathologiquement synchrone et oscillatoire, et les seconds stimuli (20) provoquent la réinitialisation de la phase de l'activité neuronale de la seconde sous-population, et les première et seconde unités de stimulation (11, 12) sont conçues de telle manière que les premier et second stimuli (20) sont administrés à des moments au moins partiellement différents, de sorte que les phases de l'activité neuronale des première et seconde sous-populations sont réinitialisées à des moments différents et que les première et seconde sous-populations présentent des phases différentes de leur activité neuronale après les réinitialisations de phase provoquées par les premier et second stimuli (20).

2. Dispositif (100 - 300) selon la revendication 1, dans lequel les première et seconde unités de stimulation (11, 12) sont conçues de telle manière que les premier et second stimuli (20) sont répétés pendant une période de stimulation avec respectivement en moyenne une fréquence $f_{stim}$ comprise entre 1 et 60 Hz.

Fig. 1

Fig. 2

14

Fig. 3A

Fig. 3B

Fig. 3C

Fig. 3D

Fig. 4

Fig. 5A

Fig. 5B

Fig. 5C

Fig. 6

Fig. 7

Fig. 8

Fig. 9

200

10 ~

11

12

13

14

16

Fig. 10

41 ~

40 ~

15 ~

Fig. 11A

41 ~

~40

15'

Fig. 11B

41 ~

~40'

15'

Fig. 11C

40 ~

Fig. 12A

40 ~

Fig. 12B

Fig. 13A

Fig. 13C

Fig. 13B

Fig. 14A

Fig. 14B

Fig. 14C

Fig. 15A

Fig. 15C

Fig. 15B

Fig. 16

Fig. 17

Fig. 19

Fig. 19A

Fig. 19B

Fig. 19C

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 20070255187 A1
- WO 2009136931 A1
- US 20070232962 A1
- CA 2623464 A1
- EP 2103288 A2